# EUROPEAN PATENT SPECIFICATION

(11) **EP 2 004 830 B1**
(45) Date of publication and mention of the grant of the patent: **06.10.2010**
(21) Application number: 07736147.5
(22) Date of filing: 29.03.2007
(51) Int. Cl.: C12N 15/82, C12N 5/10, A01H 5/00

(54) **PLANT GENE PROMOTER AND ITS USE**
PFLANZENGENPROMOTOR UND DESSEN VERWENDUNG
PROMOTEUR DE GENES VEGETAUX ET SON UTILISATION

(30) Priority: 11.04.2006 US 790777 P
(43) Date of publication of application: 24.12.2008
(73) Proprietor: State of Israel, Ministry of Agriculture & Rural Development, Agricultural Research Organization (A.R.O.), Volcani Center, 50250 Bet-Dagan (IL)
(72) Inventor: GRANOT, David, 92106 Jerusalem (IL); SWARTZBERG, Dvora, 49651 Petach-Tikva (IL); GERMAN, Marcelo, Newark, Delaware 19711 (US)
(74) Representative: Fichter, Robert Arno
(86) International application number: PCT/IL2007/000407
(87) International publication number: WO 2007/116394

(56) References cited:
- WO-A-96/26283
- WO-A-96/40949
- KARNI LEAH ET AL: "Fructokinase and hexokinase from pollen grains of bell pepper (Capsicum annuum L.): Possible role in pollen germination under conditions of high temperature and CO2 enrichment." ANNALS OF BOTANY (LONDON), vol. 90, no. 5, November 2002 (2002-11), pages 607-612, XP002451443 ISSN: 0305-7364 cited in the application
- GERMAN MARCELO A ET AL: "LeFRK4, a novel tomato (Lycopersicon esculentum Mill.) fructokinase specifically expressed in stamens" PLANT SCIENCE (SHANNON), vol. 163, no. 3, September 2002 (2002-09), pages 607-613, XP002451444 ISSN: 0168-9452 cited in the application
- GERMAN MARCELO A ET AL: "Cloning, expression and characterization of LeFRK3, the fourth tomato (Lycopersicon esculentum Mill.) gene encoding fructokinase." PLANT SCIENCE (OXFORD), vol. 166, no. 2, February 2004 (2004-02), pages 285-291, XP002451445 ISSN: 0168-9452 cited in the application
- PRESSMAN ETAN ET AL: "The effect of heat stress on tomato pollen characteristics is associated with changes in carbohydrate concentration in the developing anthers." ANNALS OF BOTANY (LONDON), vol. 90, no. 5, November 2002 (2002-11), pages 631-636, XP002451446 ISSN: 0305-7364 cited in the application
- ALONI BENY ET AL: "The effect of high temperature and high atmospheric CO2 on carbohydrate changes in bell pepper (Capsicum annuum) pollen in relation to its germination" PHYSIOLOGIA PLANTARUM, vol. 112, no. 4, August 2001 (2001-08), pages 505-512, XP002451447 ISSN: 0031-9317 cited in the application
- IMIN NIJAT ET AL: "Effect of early cold stress on the maturation of rice anthers" PROTEOMICS, vol. 4, no. 7, July 2004 (2004-07), pages 1873-1882, XP002451448 ISSN: 1615-9853 cited in the application
- DAI N ET AL: "OVEREXPRESSION OF ARABIDOPSIS HEXOKINASE IN TOMATO PLANTS INHIBITS GROWTH, REDUCES PHOTOSYNTHESIS, AND INDUCES RAPID SENESCENCE" PLANT CELL, AMERICAN SOCIETY OF PLANT PHYSIOLOGISTS, ROCKVILLE, MD, US, vol. 11, July 1999 (1999-07), pages 1253-1266, XP000827859 ISSN: 1040-4651 cited in the application
- FIRON N ET AL: "Pollen grains of heat tolerant tomato cultivars retain higher carbohydrate concentration under heat stress conditions" SCIENTIA HORTICULTURAE (AMSTERDAM), vol. 109, no. 3, July 2006 (2006-07), pages 212-217, XP005519786 ISSN: 0304-4238

## Description

### FIELD OF THE INVENTION

This invention relates to a plant gene promoter and its use.

### REFERENCES

The following references are referred to in the specification and are identified in the text by their respective numbers. Mention of the reference does not necessarily indicate that it is relevant to the patentability of the invention.
1. Imin N, Kerim T, Rolfe BG, Weinman JJ (2004) Effect of early cold stress on the maturation of rice anthers. Proteomics 4: 1873-1882;
2. Aloni B, Peet M, Pharr M, Karni L (2001) The effect of high temperature and high atmospheric CO2 on carbohydrate changes in bell pepper (Capsicum annuum) pollen in relation to its germination. Physiol Plant 112: 505-512;
3. Pressman E, Peet MM, Pharr DM (2002) The effect of heat stress on tomato pollen characteristics is associated with changes in carbohydrate concentration in the developing anthers. Ann Bot (Lond) 90: 631-636;
4. German MA, Asher I, Petreikov M, Dai N, Schaffer AA, Granot D (2004) Cloning, expression and characterization of LeFRK3, the fourth tomato (Lycopersicon esculentuni Mill.) gene encoding fructokinase. Plant Science 166: 285-291
5. Kandel-Kfir M, Damari-Weissler H, German MA, Gidoni D, Mett A, Belausov E, Petreikov M, Adir N, and Granot D (2006). Two newly identified membrane-associated and plastidic tomato HXKs: characteristics, predicted structure and intracellular localization. Planta 224, 1341-1352.
6. German MA, Dai N, Chmelnitsky I, Sobolev I, Salts Y; Barg R, Schaffer AA, Granot D (2002) LeFRK4, a novel tomato (Lycopersicon esculentum Mill.) fructokinase specifically expressed in stamens. Plant Science 163: 607-613;
7. Karni, L and Aloni, B (2002) Fructokinase and hexokinase from pollen grains of bell pepper (Capsicum annuum L.): possible role in pollen germination under conditions of high temperature and CO2 enrichment; Annals of Botany, v. 90 (5):607-612;
8. Dai N, Schaffer A, Petreikov M, Shahak Y, Giller Y, Ratner K, Levine A, Granot D (1999) Overexpression of Arabidopsis hexokinase in tomato plants inhibits growth, reduces photosynthesis, and induces rapid senescence. Plant Cell 11: 1253-1266.

### BACKGROUND OF THE INVENTION

The main factor responsible for the reduction of tomato crop yields under elevated temperatures is the impairment of pollen development and germination. This factor is known as *high temperature stress* (HTS). The flowering phases most sensitive to high temperatures are meiosis (8-9 days before anthesis) and fertilization (1-3 days after anthesis). In *Brassica,* pollen from plants exposed to four days of HTS (35°C) had lower *in vitro* germination rates (17.5%) than pollen from control grown plants (59.2%). The lower germination rate was regardless of whether *in vitro* germination was carried out at 23°C or 35°C. Similar findings indicating that the male reproductive organs are the most sensitive to heat stress were reported for other plant species as well.

Although male sterility due to HTS has been observed in numerous plants, the biochemical or developmental pathways affected remain unknown. Similarly, while much is known about the response of vegetative tissues to environmental stress, very little information is available with respect to the pollen's ability to mount a defensive response. A comparative analysis of the global pollen protein expression profile in the presence or absence of stress is one approach that can help address these shortcomings. To date, there are still very few comprehensive analyses specific to the pollen proteome and only one such analysis deals with temperature stress [1]. In this study the global protein expression profile of rice anthers was analyzed under cold stress and 70 protein spots were found to be differentially displayed. The little evidence so far seems to indicate that pollen grains not only do not induce the proteins normally found in stressed vegetative tissues, but that the detrimental effects occur mainly at the translational and post-translational level.

Carbohydrates play a critical role in pollen development, germination and fertilization, serving as nutrients for metabolic, structural, storage and perhaps signaling functions. In many plants including tomato and *Brassica,* the transported sugar is mainly sucrose. An unloading pathway of sucrose via the apoplastic space is mandatory for symplastically isolated cells, such as developing pollen, that receive their carbohydrate supply from the tapetum layer and the surrounding locular fluid. The transported sucrose is released from the sieve elements of the phloem into the anther wall layers and the tapetum apoplast, probably via a sucrose transporter. The released sucrose molecules may follow either or both of the following routes: 1. Enter the cytosol of the pollen sink cells and be cleaved by sucrose synthase (SuSy) into fructose and UDP-glucose (UDPG); 2. Irreversibly hydrolyzed into glucose and fructose by an extra-cellular (apoplastic) invertase - ionically bound to the cell wall. The resulting hexose monomers, glucose and fructose, are then taken up into the sink cells by hexose transporters.

Pollen germination and tube growth are also highly dependent on the availability of sugars, since the main metabolic activity during this process is the biosynthesis of polymers that will form the elongating cell wall. While the volume of the protoplast does not change significantly during pollen tube elongation since it moves forward, the cell wall forms an immobile tube which continuously expands at the apex. Given that a single pollen tube has to achieve a total length of about a centimeter in tomato and *Brassica* pistils within a short time, the supply of cell wall precursors needs to be uninterrupted and extremely rapid. To support this high level of carbohydrate synthesis pollen tubes use both stored reserves which can include sucrose, starch, phytic acid and lipids, depending on the species and external sources of sugar. In *in vitro* growing pollen tubes, the need for external carbon sources was shown to increase with time as internal stores were depleted. Efficient sugar metabolism is therefore crucial for the success of the fertilization process.

In a number of plant species, stress-induced impairment of male gametophyte development is preceded by disturbances in carbohydrate metabolism in the anther and the developing pollen [2]. In tomato, HTS causes a reduction in both starch levels of developing immature tomato pollen and soluble sugar concentration of mature pollen grains. This was accompanied by a decrease in the number of pollen grains produced, an elevated number of non-viable pollen and a decrease in the capacity of the viable pollen to germinate [3].

Both sucrose synthase (SuSy) and invertase cleave sucrose to produce the hexose sugars, glucose and fructose, that must be phosphorylated by hexose phosphorylating enzymes (hexose kinases) before they can be further metabolized. Hexose phosphorylating enzymes are characterized by their particular affinity to various sugars. Hexokinase (HXK) phosphorylates glucose and fructose, whereas fructokinase (FRK) phosphorylates only fructose. The affinity of FRKs to fructose are one to two orders of magnitude higher than that of HXKs to fructose. The phosphorylated sugars (hexose phosphates) may enter glycolysis or the pentose phosphate pathway, or be converted and stored as starch. Uridine diphosphate glucose (UDPG), produced by cleavage of sucrose by SuSy, may also be used for cell wall, cellulose or starch synthesis.

Four HXK genes and four FRK genes (*LeFRK1-4*) were identified in tomato plants [4, 5]. All four HXK and three of the FRK genes (*LeFRK1-3*) are expressed at different levels in all plant tissues [4, 5]. However, the fourth recently cloned fructokinase, *LeFRK4,* is expressed exclusively in anthers and pollen [6] (mainly in mature and germinating pollen but also in developing pollen) at levels 100 times higher than any of the other HXK and FRK genes.

Karni, L and Aloni, B. [7] investigated changes in fructokinase (FRK) and hexokinase activities in pepper flowers during their development, and studied the possible roles of these enzymes in determining pollen germination capacity under high temperature and under CO₂ enrichment, previously shown to modify sugar concentrations in pepper pollen. Their results suggested that pollen and anther FRK may play a role in the regulation of pollen germination, possibly by providing fructose-6-phosphate for glycolysis, or through conversion to UDPG to support the biosynthesis of cell wall material for pollen tube growth.

### SUMMARY OF THE INVENTION

Disclosed is an isolated polynucleotide sequence comprising one of the following: (a) the LeFRK4 promoter; and (b) a functional part thereof, being a modified LeFRK4 promoter in which one or more of the nucleotide bases have been substituted or deleted, or one or more nucleotide bases have been added thereto, wherein the specific promoter activity of the modified promoter is substantially the same as that of the LeFRK4 promoter.

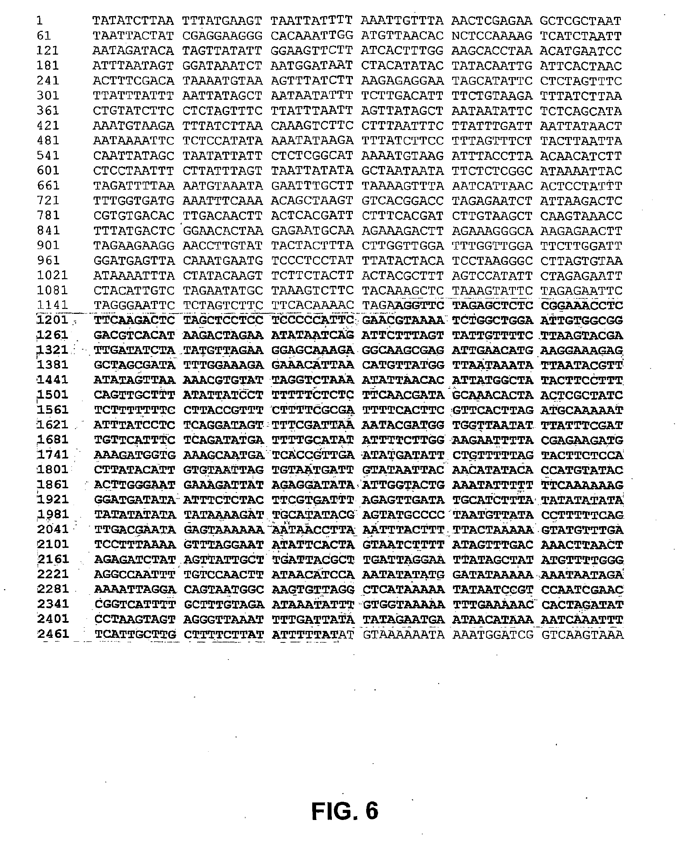

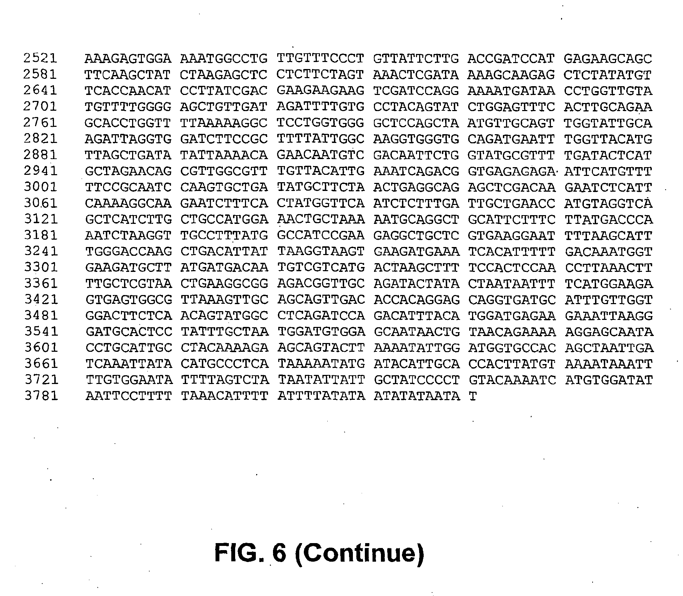

The term *"LeFRK24 promoter"* includes the sequence consisting of nucleotides 1 to 2488 (SEQ. ID.NO:1) of the sequence shown in Fig. 6.

The term also refers to nucleotides 1175 to 2488 (SEQ.ID.NO: 2) of the sequence shown in Fig. 6. It should be noted that the 14bp sequence at the beginning of the LeFRK4 gene (bp 2489-2502 in Fig. 6) is apparently not expressed, and may be a 5' UTR sequence. The promoter nucleotide sequence 1175 to 2488 (SEQ.ID.NO: 2) has been deposited at GenBank on March 19, 2006, and has been accorded accession number DQ453118.

The term *"specific promoter activity"* within the context of the LeFRK4 promoter includes at least the following features:
1. the ability of the promoter to drive the expression of a gene, preferably a gene which may encode a sugar kinase, and in particular, hexokinase or fructokinase;
2. the promoter is active primarily, and preferably exclusively, in the anther and pollen portions of the plant.

The term *"substantially the same"* within the context of a modified LeFRK4 promoter means that the modified promoter has a specific promoter activity having at least one, and preferably both of its features, defined above, at a level of at least 80%, more preferably at least 85%, still more preferably at least 90%, even more preferably at least 95%, most preferably at least 98% of the activity of the unmodified, native promoter.

Also included in the invention is a vector, preferably an expression vector, comprising the promoter of the invention. Various expression vectors active in plant cells are well known to the skilled man of the art, e.g. PVX, pJLX, pBI121, pART7/27, pRT104, etc. Various heterologous nucleic acid sequences or genes may be inserted into the vector so as to be operably linked to and under control of the promoter. In a preferred embodiment, the heterologous gene encodes a sugar kinase, for example hexokinase, or fructokinase.

A further embodiment of the invention includes a host cell transformed by the vector of the invention. In a preferred embodiment, the host cell is a plant cell, preferably a pollen or an anther cell. A still further embodiment of the invention is a transgenic plant comprising the host cell of the invention.

The promoter as disclosed herein may be used in various applications in plant genetics. One of the applications is described in detail below.

Other possible applications are: (1) causing male sterility by specific expression of various genes (such as RNAses, proteases, toxins or other genes) that may disrupt pollen or anther development. Male sterility is a desirable trait for the production of hybrid seeds and may save laborious sterilization methods currently used in company seeds; and (2) elimination of pollen development to prevent or reduce allergenic pollen components or allergic effects caused by pollen.

In a second aspect of the invention, there is provided a method for protecting a plant from high temperature stress (HTS), comprising transforming the plant with a polynucleotide sequence, wherein said polynucleotide sequence comprises a nucleotide sequence encoding hexokinase or a fructokinase under the control of the LeFRK4 promoter, thereby producing a transformed plant having improved tolerance to HTS.

In a preferred embodiment, the specific promoter comprises the LeFRK4 promoter. In another preferred embodiment, the sugar kinase is a hexokinase or a fructokinase. In a still other preferred embodiment, the plant is tomato, cucumber, peppers, zucchini, maize, cotton, flax, wheat, rice, eggplant, melon or *Brassica,* or any other plant susceptible to HTS.

Also disclosed is a method for causing male sterility comprising transforming the plant with a polynucleotide sequence, wherein said polynucleotide sequence comprises a nucleotide sequence capable of disrupting pollen or anther development under the control of an anther and/or pollen specific promoter, thereby causing male sterility, the specific promoter comprises the LeFRK4 promoter.

### BRIEF DESCRIPTION OF THE DRAWINGS

In order to understand the invention and to see how it may be carried out in practice, it will now be described with reference to the accompanying drawings, in which:

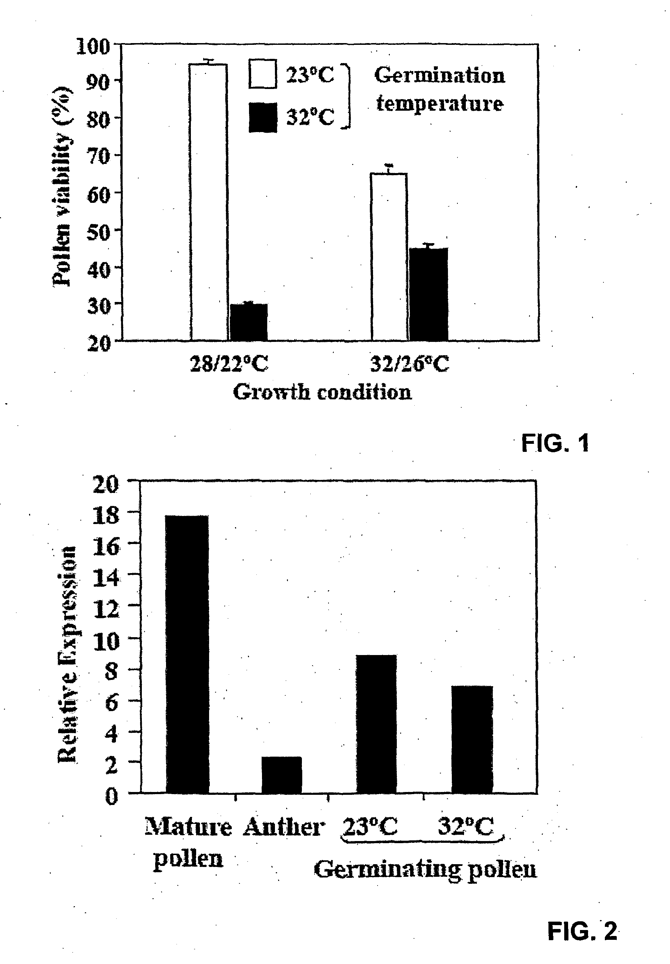

**Fig. 1** is a bar graph showing the effect of HTS on pollen development and germination. Pollen of tomato plants grown for several weeks at either normal (28°C/22°C - day/night) or high (32/26°C) temperatures were collected and germinated at low (23°C) and high (32°C) temp. Pollen viability was scored under the microscope following staining with Alexander reagent as described in Pressman E, Moshkovitch H, Rosenfeld K, Shaked R, Gamliel B and Aloni B (1998), Influence of low night temperatures on sweet pepper flower quality and the effect of repeated pollinations, with viable pollen, on fruit setting. Journal of Horticultural Science and Biotechnology 73: 131-136. Viable pollen includes germinating and stained grains;
**Fig. 2** is a bar graph showing expression of 35S::AtHXK1 in anther and pollen. The expression of the *Arabidopsis* hexokinase *AtHXh1* in tomato plants under 35S. promoter was determined in mature pollen, anther (without pollen) and germinating pollen. The expression was determined by real time PCR with *AtHXK1* specific primers, and normalized to the expression of a house keeping gene cyclophilin (similar results were obtained upon normalization with rRNA);

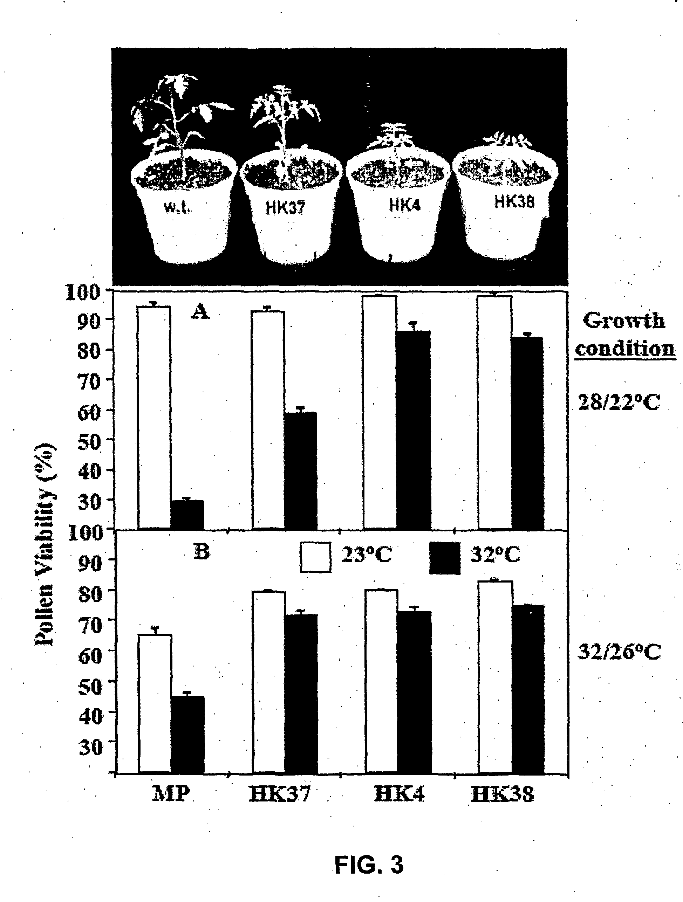

**Fig. 3** includes photographs and bar graphs and shows the effect of HTS on pollen germination. Control tomato plants (MP, w.t.) and independent isogenic transgenic tomato lines overexpressing *AtHXK1* (HK37, HK4 and HK38) were grown for several weeks at either normal (A) (28°C/22°C - day/night) or high (B) (32/26°C) temperatures. HK4 and HK38 plants had high expression of *AtHXK1* whereas HK37 plants had a moderate expression level [8]. Pollen were collected and germinated at low (23°C) and high (32°C) temp. Pollen viability was scored as described in Fig. 1;

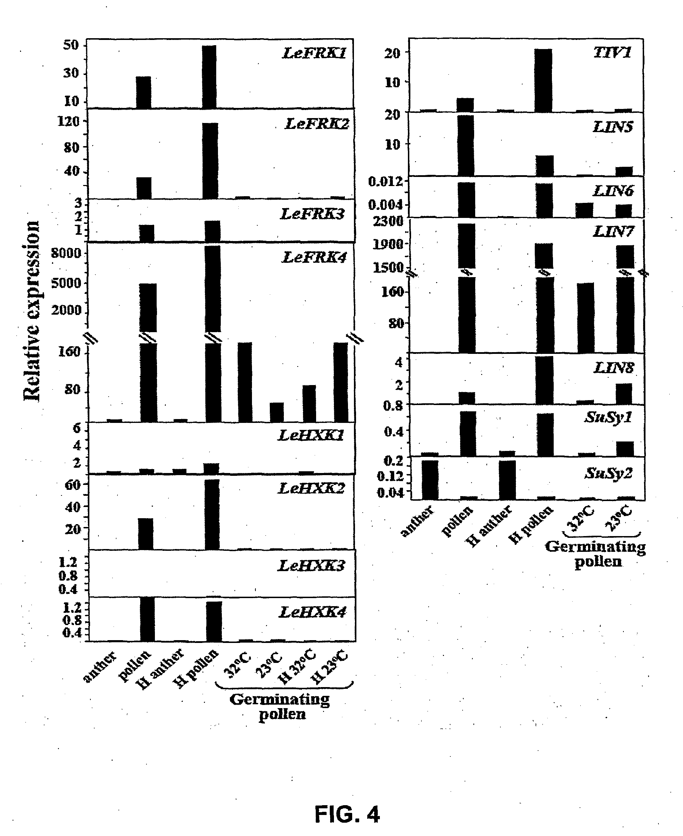

**Fig. 4** shows bar graphs illustrating relative expression of various sugar metabolizing genes in tomato anther and pollen. *LeFRK* are fructokinases, *LeHXK* are hexokinases, *TIV1, Lin5-8* are invertases, *SuSy* are sucrose synthases and *LeGLT* is a plastidic glucose transporter. Gene expression was determined by real time PCR with gene specific primers and was normalized to cylcophilin as a house keeping gene. Samples with the letter H were taken from plants grown at HTS (32°/26°C);

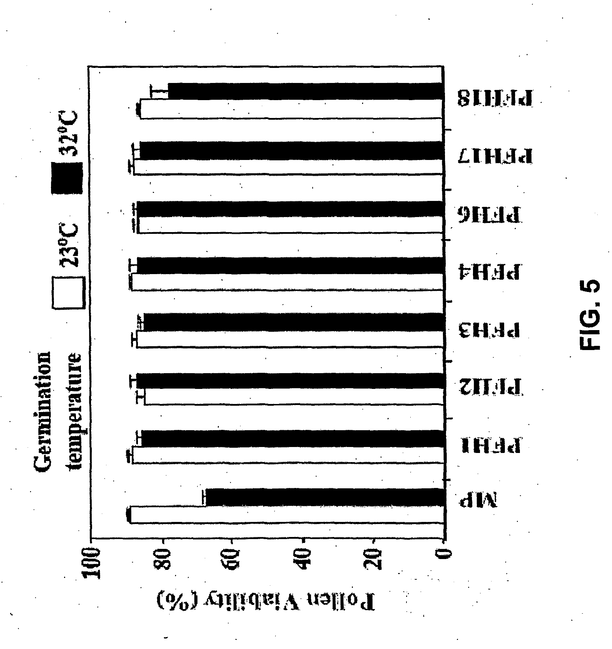

**Fig. 5** is a bar graph showing the effect of *P_{LeFRK4}::AtHXK1* on pollen germination. Control tomato plants (MP) and original (To) independent isogenic transgenic tomato lines overexpressing *P_{LeFRK4}::AtHXK1* (assigned PFH lines 1-8) were grown at normal (28°C/22°C - day/night) temperatures. Pollen were collected and germinated at low (23°C) and high (32°C) temp. Pollen viability was scored as described in Fig. 1; and
**Fig. 6** shows the nucleotide sequence of the *LeFRK4* promoter comprising 2488 bp, followed by the cDNA sequence of the *LeFRK4* gene (bp 2489-3821). The promoter was sequenced from BAC colonies using appropriate primers, as indicated below.

### DETAILED DESCRIPTION OF EXEMPLARY EMBODIMENTS

| **List Of Abbreviations** | | |
|---|---|---|
| CaMV | - | Cauliflower mosaic virus |
| FRK | - | Fructokinase |
| Fru6p | - | Fructose 6-phosphate |
| Glc6P | - | Glucose 6-phosphate |
| HXK | - | Hexokinase |
| *LeHXK1,2,3,4* | - | *L. esculentum hexokinase 1, 2, 3 and 4* |
| *LeFRK1,2,3,4* | - | *L. esculentum fructokinase 1,* 2, 3 and 4 |
| PCR | - | Polymerase Chain Reaction |
| SuSy | - | Sucrose Synthase |
| UDP-glucose | - | Uridyl diphosphate glucose |

| | |
|---|---|
| **List of growth media** | |
| **1/2 x MSO:** | 2.3gr/lit Murashige and Skoog (MS) salts |
| | 3% sucrose |
| | 0.8% agar |
| | pH 5.8 |
| **Do:** | 4.3gr/lit MS salts |
| | 3% glucose |
| | 1mg/lit zeatin |
| | 0.8% agar |
| | pH 5.8 |
| **D1**: | 4.3gr/lit MS salts |
| | 3% glucose |
| | 1mg/lit zeatin |
| | 0.8% agar |
| | 100mg/lit kanamycin |
| | 300mg/lit carbenecelin |
| | pH 5.8 |
| **MSR:** | 4.3gr/lit MS salts |
| | 3% sucrose |
| | 1mg/lit IBA |
| | 0.8% agar |
| | pH 5.8 |
| **LB:** | 10gr/lit tryptone |
| | 5gr/lit yeast extract |
| | 5gr/lit NaCl |
| | 15gr/lit agar |

| | |
|---|---|
| **Primers** | |
| 1. | LeHXK1 fw: GGACTTGCTGGGAGAGGAGT (SEQ.ID.NO: 3); |
| 2. | LeHXK1 rev: AAGGTACATTGAATGAGAGGCA (SEQ. ID NO:4); |
| 3. | LeHXK2 fw: GTCCTCCCATCTTCCCTTG (SEQ. ID NO:5); |
| 4. | LeHXK2 rev: CCCAAGTACATACCAGAACAT (SEQ. ID NO:6); |
| 5. | LeHXK3 fw: GCGATATTATCACCTCTCGTG (SEQ. ID NO:7); |
| 6. | LeHXK3 rev: CTGCTTCTCTCCGTCTTTAAA (SEQ. ID NO:8); |
| 7. | LeHXK4 fw: GCTGAGGACACCTGATATATG (SEQ. ID NO:9); |
| 8. | LeHXK4 rev: GATCGGATTTTACCCCAGCTA (SEQ. ID NO:10); |
| 9. | LeFRK1 fw: CTCCGTTACATATCTGATCCT (SEQ. ID NO:11); |
| 10. | LeFRK1 rev: GACAGCATTGAAGTCACCTT (SEQ. ID NO:12); |
| 11. | LeFRK2 fw: TTGTTGGTGCCCTTCTAACCA (SEQ. ID NO:13); |
| 12. | LeFRK2 rev: ACGATGTTTCTATGCTCCTCCCT (SEQ. ID NO:14); |
| 13. | LeFRK3 fw: TTACGGAGCCATGCAAATCAG (SEQ. ID NO:15); |
| 14. | LeFRK3 rev: GCCACAATGGAAGCCCTCAATT (SEQ. ID NO:16); |
| 15. | LeFRK4 fw: GGTGATGCATTTGTTGGTGGAC (SEQ. ID NO:17); |
| 16. | LeFRK4 rev: GCTGTGGCACCATCCAATATTT (SEQ. ID NO:18); |
| 17. | TIV1 fw: GAAGTGGACAAAGTCGCGCTT (SEQ ID NO:19); |
| 18. | TIV1 rev: CTGGCGTTAGCTCAGATAGCGT (SEQ. ID NO:20); |
| 19. | Lin5 fw: GAACTGAGTTTCGCGATCCAACT (SEQ. ID N021); |
| 20. | Lin5 rev: TGAAGTGGATGTTGGGCTTTG (SEQ. ID NO:22); |
| 21. | Lin6 fw: ACCCAAAAGGAGCAACATGG (SEQ. ID NO:23); |
| 22. | Lin6 rev: CCTGGTAAGATTGTGGCTGACC (SEQ. ID NO:24); |
| 23. | Lin7 fw: TTGTTTGGGCTCATTCCGTT (SEQ. ID NO:25); |
| 24. | Lin7 rev: CCGGTGTACAAGATGACTGGCT (SEQ. ID NO:26); |
| 25. | Lin8 fw: AACCCGCAATCTATCCATCCA (SEQ. ID NO:27); |
| 26. | Lin8 rev: TCCGCTGGGATTGCATAGTTT (SEQ. ID NO:28); |
| 27. | SuSy1 fw: TTCACCATGGCAAGATTGGAC (SEQ. ID NO:29); |
| 28. | SuSy1 rev: TCTCTGCCTGCTCTTCCAAGTC (SEQ. ID NO:30); |
| 29. | SuSy2 fw: CATCACCAGCACATTCCAGGA (SEQ. ID NO:31); |
| 30. | SuSy2 rev: AGCTCCAGGTGAGACGATGTTG (SEQ. ID NO:32); |
| 31. | LeGT fw: GGGTTGCTGAGGTTATGAGTGATT (SEQ. ID NO:33); |
| 32. | LeGT rev: AGCTGCACCAACGCTAACAA (SEQ. ID NO:34); |
| 33. | Cyclo fw: CGTCGTGTTTGGACAAGTTG (SEQ. ID NO:35); |
| 34. | Cyclo rev: CCGCAGTCAGCAATAACCA (SEQ. ID NO:36); |
| 35. | Fk4102: ATCGAGTTTACTAGAAGAGGA (SEQ. ID NO:37); |
| 36. | Fk4128: GGTGAACATATAGAGCTCTTGCTT (SEQ. ID NO:38); |
| 37. | Fkpro656: GCATATCAACTCTAAATCACGAAG (SEQ. ID NO:39); |
| 38. | Fkpro659: GATGCATATCAACTCTAAATCACG (SEQ. ID NO:40); |
| 39. | Fkpro776: TGAAGAAGACTAGAGGAATTCCCT (SEQ. ID NO:41); |
| 40. | LeFRK4pro fw: AGGTTCTAGAGCTCTCCGGAAA (SEQ. ID NO:42); |
| 41. | LeFRK4pro rev: GATCGGTCAAGAATAACAGGG (SEQ. ID NO:43); |
| 42. | LeFRK4pro fw: ACCACTAGATATCCTAAGTAGTA (SEQ. ID NO:44); |
| 43. | AtHXK1 fw: GCGGGAAGCAAGAGCGTGTT (SEQ. ID NO:45); |
| 44. | AtHXK1 rev: CTCCTCGGGTTGCTATGATG (SEQ. ID NO:46); |

### Examples

### 1. The effect of HTS on pollen development and germination

The effect of HTS on both pollen development and germination is demonstrated in Figure 1. Pollen of tomato plants grown at normal temperatures (28/22°C day/night) had good viability upon germination at 23°C and markedly reduced viability upon germination at 32°C, indicating a negative effect of HTS on pollen germination. Pollen of plants grown at high temperatures (32/26°C) also displayed lower viability when germinated at 23°C indicating a persisting negative effect of HTS on pollen development.

### 2. Over-expression of AtHXK1 improves development and germination of tomato pollen under HTS

To investigate the importance of hexose phosphorylation in determining pollen competence to germinate, pollen germination was analyzed for tomato plants overexpressing the Arabidopsis hexokinase gene (*AtHXK1*) under the control of the CaMV 35S promoter (*35S::AtHK1*) [8]. Although previous reports claimed that this promoter had only negligible expression in tomato pollen, it was found that the *35S::AtHXK1* construct is expressed in pollen, in accordance with published results for tomato and other plant species [Duck NB, Folk WR (1994) Hsp70 heat shock protein cognate is expressed and stored in developing tomato pollen. Plant Mol Biol 26: 1031-1039], as well as in empty tomato anthers and in pollen germinating at either 23°C or 32°C (Figure 2). Since the CaMV 35S promoter expresses in all plant organs, expression of the Arabidopsis *AtHXK1* under this promoter accelerated leaf senescence and inhibited growth [8]. Nevertheless, pollen of tomato plants overexpressing *AtHXK1* had a significantly higher viability when either grown or germinated at high (32°C) temperatures in direct correlation with the occurrence of *AtHXK1* expression (Figure 3). Although HK37, HK4 and HK38 plants have less photosynthetic capacity, they were still able to set fruits upon growth at HTS (32/28°C) unlike the control plants. These results strongly suggest that hexose phosphorylation in anther and pollen may affect the germination potential of pollen and highlights the need to use anther- and pollen-specific promoters to drive the expression of the *AtHXK1.*

### 3. LeFRK4 is highly expressed in mature and germinating tomato pollen

Recently, a fructokinase gene, *LeFRK4,* was cloned which is exclusively expressed in stamens [6]. The expression of *LeFRK4* was analyzed in developing and germinating pollen relative to the other three FRK genes (*LeFRK1,* 2 & 3), the four known HXK genes (*LeHXK1-4*), the 5 invertase genes (TIV1, *LIN5-8*) and the two SuSy genes (*SuSy1-2*). It was found that *LeFRK4* is by far the most abundantly expressed gene in both mature and germinating tomato pollen (Figure 4). *LeFRK4* is also expressed in developing pollen grains and in other parts of the anther, although at a lower level (not shown). The *LeFRK4* promoter should therefore be a good candidate to drive anther- and pollen-specific expression.

### 4. Isolation of LeFRK4 promoter and its use to drive expression of AtHXK1 and LeFRK1

The preferred promoter region of *LeFRK4* (SEQ.ID.NO: 2) was isolated and joined to *AtHXK1* and to *LeFRK1* to obtain *P_{LeFRK4}::AtHXK1* and *P_{LeFRK4}::LeFkK1* constructs. To address the question of which of the two AtHXK1 activities, glucose or fructose phosphorylation, contributes to HTS resistance, HTS resistance of plants expressing *AtHXK1* or *LeFRK1* under the control of the *LeFRK4* promoter should be compared. To reduce the possibility of cosuppression, *LeFRK1* should be used, whose normal expression in anther and pollen is relative low. *LeFRK1* should be preferred over *LeFRK2* and *LeFRK3* since *LeFRK1,* unlike the latter enzymes (but like *LeFRK4*), is not repressed by fructose and perhaps could further enhance fructose phosphorylation.

The *P_{LeFRK4}::AtHXK1* and *P_{LeFRK4}::LeFRK1* constructs have been used to generate about thirty transgenic tomato plants per construct. Initial analysis of To plants expressing *P_{LeFRK4}::AtHXK1* has shown that independent transgenic plants exhibit high pollen viability upon germination at high temperature (Figure 5). In addition, unlike the growth inhibition observed with HK plants (plants expressing *AtHXK1* under the CaMV 35S promoter), plants expressing *P_{LeFRK4}::AtHXK1* exhibited entirely normal growth and development (not shown).

## Claims

1. An isolated polynucleotide sequence as set forth in SEQ ID NO: 1 or 2.

2. A vector comprising the polynucleotide sequence of claim 1 operably linked to a heterologous gene which encodes a sugar kinase selected from the group consisting of hexokinase and fructokinase.

3. A host cell comprising the vector of claim 2.

4. A transgenic plant comprising the host cell of claim 3.

5. A method of increasing pollen viability of a plant at temperatures that reduce pollen viability of said plant, the method comprising transforming the plant with a polynucleotide sequence, wherein said polynucleotide sequence comprises a nucleotide sequence encoding a sugar kinase selected from the group consisting of hexokinase and fructokinase under the control of the isolated polynucleotide of claim 1, thereby producing a transformed plant which exhibits increased pollen viability at said temperatures.

6. The method of claim 5, wherein said temperatures are 32°C/26°C day/night, respectively.

7. The method of claim 5 or 6 wherein said plant is selected from tomato, cucumber, peppers, zucchini, maize, cotton, flax, wheat, rice, eggplant, melon and *Brassica.*

8. The vector of claim 2 or the method of claim 5, 6 or 7, wherein said sugar kinase is hexokinase.

9. The vector of claim 2 or the method of claim 5, 6 or 7, wherein said sugar kinase is fructokinase.

## Patentansprüche

1. Isolierte Polynucleotidsequenz, wie in SEQ ID NR.: 1 oder 2 dargelegt.

2. Vektor, der die Polynucleotidsequenz nach Anspruch 1 umfasst und funktionsfähig mit einem heterologen Gen gekoppelt ist, das eine Zuckerkinase codiert, die aus der Gruppe bestehend aus Hexokinase und Fructokinase ausgewählt ist.

3. Wirtszelle, die den Vektor nach Anspruch 2 umfasst.

4. Transgene Pflanze, welche die Wirtszelle nach Anspruch 3 umfasst.

5. Verfahren zur Erhöhung der Lebensfähigkeit von Pollen einer Pflanze bei Temperaturen, welche die Lebensfähigkeit der Pollen der Pflanze verringern, wobei das Verfahren ein Transformieren der Pflanze mit einer Polynucleotidsequenz umfasst, wobei die Polynucleotidsequenz eine Nucleotidsequenz umfasst, die eine Zuckerkinase, die aus der Gruppe bestehend aus Hexokinase und Fructokinase ausgewählt ist, unter der Kontrolle des isolierten Polynucleotids nach Anspruch 1 codiert, um **dadurch** eine transformierte Pflanze zu erzeugen, welche eine erhöhte Lebensfähigkeit der Pollen bei den Temperaturen aufweist.

6. Verfahren nach Anspruch 5, wobei die Temperaturen 32 °C/26 °C am Tag bzw. in der Nacht betragen.

7. Verfahren nach Anspruch 5 oder 6, wobei die Pflanze aus Tomate, Gurke, Paprikas, Zucchini, Mais, Baumwolle, Flachs, Weizen, Reis, Aubergine, Melone und *Brassica* ausgewählt ist.

8. Vektor nach Anspruch 2 oder Verfahren nach Anspruch 5, 6 oder 7, wobei es sich bei der Zuckerkinase um Hexokinase handelt.

9. Vektor nach Anspruch 2 oder Verfahren nach Anspruch 5, 6 oder 7, wobei es sich bei der Zuckerkinase um Fructokinase handelt.

## Revendications

1. Séquence de polynucléotides isolée telle que présentée dans SEQ ID NO : 1 ou 2.

2. Vecteur comprenant la séquence de polynucléotides de la revendication 1, liée de manière opérationnelle à un gène hétérologue qui code pour une kinase de sucre choisie dans le groupe consistant en l'hexokinase et la fructokinase.

3. Cellule hôte comprenant le vecteur de la revendication 2.

4. Plante transgénique comprenant la cellule hôte de la revendication 3.

5. Procédé d'augmentation de la viabilité du pollen d'une plante à des températures qui réduisent la viabilité du pollen de ladite plante, le procédé comprenant la transformation de la plante avec une séquence de polynucléotides, où ladite séquence de polynucléotides comprend une séquence de polynucléotides codant pour une kinase de sucre choisie dans le groupe consistant en l'hexokinase et la fructokinase sous le contrôle du polynucléotide isolé de la revendication 1, produisant ainsi une plante transformée qui affiche une viabilité du pollen auxdites températures.

6. Procédé selon la revendication 5, dans lequel lesdites températures sont de 32 °C/26 °C jour/nuit, respectivement.

7. Procédé selon la revendication 5 ou 6, dans lequel ladite plante est choisie parmi la tomate, le concombre, les poivrons, la courgette, le mais, le coton, le lin, le blé, le riz, l'aubergine, le melon et *Brassica*.

8. Vecteur selon la revendication 2 ou procédé selon la revendication 5, 6 ou 7, dans lequel ladite kinase de sucre est l'hexokinase.

9. Vecteur selon la revendication 2 ou procédé selon la revendication 5, 6 ou 7, dans lequel ladite kinase de sucre est la fructokinase.
